**Europäisches Patentamt**

**European Patent Office**

**Office européen des brevets**

(19)

(11) Publication number : **0 396 232 B1**

(12) # EUROPEAN PATENT SPECIFICATION

(45) Date of publication of patent specification :
**02.03.94 Bulletin 94/09**

(51) Int. Cl.⁵ : **A61K 7/16, A61K 7/26**

(21) Application number : **90302606.0**

(22) Date of filing : **12.03.90**

(54) **Oral rinse compositions.**

(30) Priority : **13.03.89 US 322659**

(43) Date of publication of application :
**07.11.90 Bulletin 90/45**

(45) Publication of the grant of the patent :
**02.03.94 Bulletin 94/09**

(84) Designated Contracting States :
**AT BE CH DE DK ES FR GB GR IT LI NL SE**

(56) References cited :
**US-A- 4 376 115**
**JOURNAL OF PERIODONTOLOGY, vol. 58, no. 9, 1987, pages 622-627; J.L. BARCZYNSKI et al.: "Viadent, ethanol, and pH effects upon gingival epithelial-like cells, in vitro"**
**CARIES RES., vol. 21, no. 3, 1987, pages 285-288; J. AFSETH et al.: "Clinical experiments with a mouthrinse containing sanguinarine chloride"**

(73) Proprietor : **VIPONT PHARMACEUTICAL, INC.**
**1625 Sharp Point Drive**
**Fort Collins Colorado 80525 (US)**

(72) Inventor : **Donohue, John J.**
**1749 Concord Drive**
**Fort Collins, Colorado (US)**
Inventor : **Harkrader, Ronald J.**
**336 VanBuren Court**
**Louisville, Colorado (US)**
Inventor : **Jones, Richard R.**
**2801 Virginia Dale Drive**
**Fort Collins, Colorado (US)**
Inventor : **Peterson, Kenneth S.**
**2620 Powell**
**Fort Collins, Colorado (US)**

(74) Representative : **Kearney, Kevin David Nicholas et al**
**KILBURN & STRODE 30 John Street**
**London, WC1N 2DD (GB)**

## Description

This invention relates to novel sanguinarine mouthrinses stabilized at a pH which provides improved uptake of sanguinaria into plaque and improved protection against acid production by salivary bacteria after a glucose challenge.

Sanguinarine canadensis is known as Bloodroot, Puccoon, Tetterwort, etc. and is a herb native to North America. The plant and its juices have been used for various purposes during pre-history as well as written history. It has been used as a natural historic folk remedy medicine. The plant has been generally used whole either undried (fresh) or dried. The usual procedure is to powder the dried plant and mix it with a carrier. This folk remedy has been tried for such things as asthma, bronchitis, dysentery, ringworm, and a substantial list of other ailments. Sanguinarine, chelerythine, and other benzophenanthridine alkaloids are known alkaloids defined as isoquinolines. Plant sources for these alkaloids fall into various species, the Papaveraceae, Fumariaceae, and Rutaceae families. Recoveries of these alkaloids from Sanguinaria Canadensis L. and Macleaya species are described in U.S. Patents 4,145,412; 4,406,881; 4,517,172; 4,590,061; 4,599,228; 4,683,133; 4,689,216; 4,767,861; and 4,769,452. Production of these alkaloids from plant tissue culture has been described in Plant Cell Reports (1988) 7:410-413.

Purification of the Benzophenanthridine alkaloids into individual alkaloids without using known chromatographic methods is also described in the foregoing U.S. Patents.

The use of an extract containing these benzophenanthridine alkaloids from Sanguinaria Canadensis as an ingredient in an oral cleaning preparation is also disclosed in the foregoing U.S. Patents.

Other uses for the extract of Sanguinaria Canadensis L. are reported as a plaque disclosing agent in U.S. Patents 4,517,172 and 4,590,061.

It is known that mouthrinses and dentifrices containing sanguinaria extract are effective anti-plaque and anti-gingivitis agents. They are also effective against oral malodor and calculus.

Early patents, U.S. Patent 209,331 and U.S. Patent 2,344,830 describe the use of sanguinaria in combination with zinc chloride. Although there is no indication that zinc chloride is needed to maintain efficacy, sanguinaria extract is typically used in combination with zinc chloride or other metallic salts, such as stannous chloride, in dentifrices, as in U.S. Patent 4,689,216. When sanguinaria extract is combined with zinc chloride in a mouthrinse vehicle the pH is usually adjusted to 3. Prior art teaches that zinc mouthrinses are adjusted to pH 3 to prevent the formation of insoluble zinc compounds such as zinc hydroxide and zinc oxychloride. See U.S. Patent 4,289,755. In addition, solutions of sanguinaria are acidified to prevent the formation of pseudo base forms of the benzophenanthridine alkaloids present in the extract. Pseudo base forms of the alkaloids present in the extract begin to form at approximately pH 5.6 (Jones, et al. J. of Natural Products, Vol. 49, No. 6, pp.1109-1111, Nov.-Dec., 1986).

In addition, the extract was adjusted to a pH of 3 to maintain chemical stability of the extract and prevent the formation of brownish to black precipitates which are assumed to be due to the condensation of tannins and to the presence of lignin-like material in the extract. It has been found that 1% solutions of sanguinaria extract filtered at pH's of 3, 4, and 5 will form additional precipitates on standing with the minimum amount of precipitate forming at pH 3. This indicates that at pH's above 3 undesirable condensation and/or polymerization begins to occur.

The alkaloid extract when made into an oral rinse, dentifrice, or oral care product is an excellent breath freshener, and also an anti-plaque and anti-gingivitis agent. When combined with zinc chloride, however, the pH of the rinse needs to be acidic (pH=3) for a number of reasons. Zinc containing rinses are generally formulated at a pH of 3 for stability purposes as set forth in U.S. Patent 4,289,755. Other patent art teaches that higher pH's are attainable through the use of glycine. See U.S. Patent 4,339,432. Rinses at low pH's, especially with zinc chloride, also taste acrid.

The present invention provides novel mouthrinses containing sanguinaria extract of superior stability and improved uptake of sanguinaria into plaque, and improved protection against acid production by salivary bacteria.

According to the present invention an oral composition contains benzophenanthridine alkaloid stabilized by the presence of an orally acceptable buffer system buffered to acid pH, preferably buffered to pH 4.0 to 5.6.

A preferred form of stable oral rinse composition according to the invention comprises from 0.01 to 0.2% by weight of benzophenanthridine alkaloid in an aqueous solution and a buffer system in a quantity sufficient to adjust the pH of said composition to a pH in the range of 4.0 to 5.6.

The buffer system is preferably citrate, acetate, tartrate, succinate, lactate, propionate or phosphate buffer system.

The said buffer system is preferably selected from the group consisting of (a) sodium citrate and citric acid, (b) sodium acetate and acetic acid, and (c) sodium tartrate and tartaric acid. The benzophenanthridine alkaloids which are useful in the composition of this invention include sanguinarine, chelerythrine, sanguilutine, chelilutine, chelirubine, and sanguirubine.

Preferably the said alkaloid is sanguinarine or chelerythrine. The composition preferably has a pH of about 4.5.

In a preferred form of the invention the said alkaloid is selected from the group consisting of water soluble salts of sanguinarine, chelerythrine, sanguilutine, chelilutine, chelirubine, and sanguirubine; and the said buffer system is selected from (a) sodium citrate and citric acid, (b) sodium acetate and acetic acid, and (c) sodium tartrate and tartaric acid.

The composition may also comprise a water-soluble zinc salt. The zinc salt is preferably zinc chloride.

Other useful zinc salts which may be included are, for example, zinc acetate, zinc gluconate, zinc sulfate, zinc salicylate, zinc tartrate, zinc lactate, zinc phenolsulfonate, and zinc carboxymethyloxysuccinate. Preferably the zinc salt is soluble in water in the amount present in the composition, although salts which are not water-soluble may also be used if they are properly suspended or emulsified. For example, see the zinc salts described in U.S. Patent 4,100,269.

The amount of zinc salt present may vary up to about 0.5% by weight e.g. 0.05 to 0.5% by weight. Preferably, if a zinc salt is used it is present in an amount of about 0.05 to 0.25% by weight.

The oral rinse compositions of this invention generally include, in addition to the benzophenanthridine alkaloid, about 50 to 95% by weight water (preferably about 75 to 90%); 5 to 25% by weight ethyl alcohol (preferably about 5 to 15%); 2 to 15% by weight of humectant e.g. glycerine or other suitable humectant; 0.1 to 3.0% surface active agent (i.e. emulsifier); 0.02 to 1.0% by weight sweetening agent; 0.05 to 0.4% by weight flavoring agents; and 0 to 0.01% by weight coloring agents.

Other humectants suitable for this application include sorbitol, mannitol, propylene glycol, polyethylene glycols used individually or as combinations thereof. The function of these humectants are as texture, body, and flavor modifiers and as solubilizers.

The type of surface active agents suitable for this application include:

Anionics, such as sodium lauryl sulfates, sodium lauryl succinates, and sodium dodecylsulfonates.

Nonionics such as Polyoxyethylene sorbitan fatty acid esters, e.g. PEG (20) sorbitan isostearate, Polysorbate 20, Polysorbate 60, Polysorbate 80 and amine oxides, e.g. cocoamine oxide;

Amphoteric such as cocoamphocarboxypropionate and alkylbetaines, e.g. cocoamido betaines.

These surface active agents are used individually or in combinations thereof.

If a surface active agent is present and if it is a nonionic block copolymer of polyoxyethylene and polyoxypropylene then it preferably has an unsaturation of less than 45 meq/mg.

The type of sweetening agents suitable for this application include aspartame, acesulfame K, xylitol, sodium cyclamate, saccharin, and some humectants such as sorbitol, and mannitol. Flavoring agents used in the compositions of this invention should be free of sulfur compounds present in small amounts in natural mint oils such as pulegone, sabadine, and mercaptans.

The buffer system which is included in the oral rinse composition of the invention is preferably capable of maintaining the pH of the compositions in the desired range of about 4.0 to 5.6. In this pH range the stability of the compositions is very good, precipitates are avoided, and good uptake of alkaloid into plaque is attained.

There are a number of buffer systems which are useful in the compositions of the invention. When no zinc salts are present in the composition the following buffer systems are useful herein:

Sodium Citrate/Citric Acid
Sodium Acetate/Acetic Acid
Sodium Tartrate/Tartaric Acid
Sodium Phosphate/Phosphoric Acid
Sodium Succinate/Succinic Acid
Sodium Lactate/Lactic Acid
Sodium Propionate/Propionic Acid.

When zinc salts are present in the composition, preferred buffer systems are as follows:

Sodium Citrate/Citric Acid
Sodium Acetate/Acetic Acid
Sodium Tartrate/Tartaric Acid.

The invention is further illustrated by means of the following examples:

EXAMPLE I

An oral rinse composition was prepared using the ingredients listed below in the amounts stated:

| Part | Ingredients | % by Weight |
|------|-------------|-------------|
| A | Purified Water | 80.25 |
| | ZnCl$_2$ | 0.20 |
| | Glycerin | 3.00 |
| | Sodium Saccharin | 0.076 |
| B | Ethyl-Alcohol 190 Proof | 10.00 |
| | Poloxamer 237 | 0.20 |
| | Polysorbate 80 | 0.20 |
| | Flavor | 0.20 |
| C | Buffer system: | |
| |    Tri Sodium Citrate 2H$_2$O | 0.28 |
| |    Citric Acid Anhydrous | 0.016 |
| D | Fluid Extract (Sanguinaria) | |
| |    1% Aqueous Solution | 3.00 |
| E | Purified Water | 2.578 |
| | | 100.00% |

pH 4.48, 4.49.

It is important to note that to achieve optimum stability of the rinse composition, the buffer system (part C) must be added to the rinse base (parts A and B) prior to the addition of the sanguinaria extract (part D) in order to avoid color changes and formation of precipitates.

Examples of other specific buffer systems which are useful in the compositions of this invention are illustrated in the following Examples II and III.

EXAMPLE II

| Part | Ingredient | % W/W |
|------|-----------|-------|
| A | Purified Water | 80.25 |
| | Zinc Chloride | 0.20 |
| | Glycerin | 3.00 |
| | Sodium Saccharin | 0.076 |
| B | Ethyl Alcohol 190 Proof | 10.00 |
| | Poloxamer 237[1] | 0.20 |
| | Polysorbate 80[2] | 0.20 |
| | Flavor Oil | 0.20 |
| C | Sodium Acetate | 1.09 |
| | Acetic Acid, Glacial | 0.10 |
| D | Sanguinaria Extract Solution, 1% | 3.00 |
| E | Purified Water | 1.684 |
| | | 100.00% |

EXAMPLE III

| Part | Ingredient | % W/W |
|---|---|---|
| A | Purified Water | 80.25 |
| | Zinc Chloride | 0.20 |
| | Glycerin | 3.00 |
| | Sodium Saccharin | 0.076 |
| B | Ethyl Alcohol 190 Proof | 10.00 |
| | Poloxamer 237[1] | 0.20 |
| | Polysorbate 80[2] | 0.20 |
| | Flavor Oil | 0.20 |
| C | Sodium Tartrate | 0.60 |
| | Tartaric Acid | 0.02 |
| D | Sanguinaria Extract Solution, 1% | 3.00 |
| E | Purified Water | 2.254 |
| | | 100.00% |

[1] Pluronic F-87, commercially available from BASF-Wyandotte is a polyoxyethylene, polyoxypropylene block polymer.

[2] Tween 80, commercially available from ICI Americas is a mixture of oleate esters of sorbitol and sorbitol anhydrides consisting predominantly of monoesters, condensed with approximately 20 moles of ethylene oxide.

The oral rinses listed above are prepared in the following manner.

The part A ingredients are added to a main mixing vessel in the order listed, 5 minutes is allowed for mixing between each addition and a 15 minutes mixing when all of Part A has been added.

The part B ingredients are added to a separate mixing vessel in the order listed, 5 minutes being allowed for mixing between each addition and a 15 minutes mixing when all of part B has been added.

Part B is then added to part A in the main mixing vessel and mixed for 15 minutes.

Part C is dry blended and added to the main mixing vessel and mixed for 15 minutes. It is important to note that to achieve optimum stability of the rinse the buffers, "Part C", must be added to the rinse base prior to the addition of the Sanguinaria Extract Solution, part D. Color changes and precipitates may occur if this process is not followed.

Part D is added to the main mix vessel, followed by Part E which is used to rinse the Part D vessel.

After the addition of Part E the entire formula is mixed for 30 minutes and then passed through a filter having nominal pore size of 0.5 $\mu$m (microns).

Another example of an oral rinse composition containing sanguinaria and zinc salt is as follows:

EXAMPLE IV

| Part | Ingredient | % W/W |
|---|---|---|
| A | Purified Water | 80.25 |
| | Zinc Chloride | 0.20 |
| | Sorbitol, 70 | 4.11 |
| | Sodium Saccharin | 0.076 |
| B | Ethyl Alcohol 190 Proof | 10.00 |
| | Poloxamer 237[1] | 0.20 |
| | Polysorbate 80[2] | 0.20 |
| | Flavor Oil | 0.20 |
| C | Tri Sodium Citrate $2H_2O$ | 0.28 |
| | Citric Acid Anhydrous | 0.016 |
| D | Sanguinaria Extract Solution, 1% | 3.00 |
| E | Purified Water | 1.468 |
| | | 100.00% |

In order to maintain a stable pH and a precipitation free rinse containing Sanguinaria in the absence of zinc, the formulation and buffer system is not limited to previously mentioned examples.

Listed below is an additional formulation example in which there are no zinc salts present.

EXAMPLE V

| Part | Ingredient | % W/W |
|---|---|---|
| A | Purified Water | 80.25 |
| | Glycerin | 3.00 |
| | Sodium Saccharin | 0.076 |
| B | Ethyl Alcohol 190 Proof | 10.00 |
| | Poloxamer 237[1] | 0.20 |
| | Polysorbate 80[2] | 0.20 |
| | Flavor Oil | 0.20 |
| C | Tri Sodium Citrate $2H_2O$ | 0.10 |
| | Citric Acid Anhydrous | 0.07 |
| D | Sanguinaria Extract Solution, 1% | 3.00 |
| E | Purified Water | 2.904 |
| | | 100.00% |

Other buffer system examples which can be substituted for the citrate buffers in Part C are as follows:
Tartrate buffers
Acetate buffers
Phosphate buffers
Succinate buffers
Lactate buffers
Propionate buffers.

The oral rinses listed above are prepared using the same procedure previously discussed.

It has been found that sanguinaria extract becomes more chemically active as the pH is adjusted above three and can react with impurities that are present in some excipients used in the mouthrinse. For example a mouthrinse composition that shows good chemical stability at a pH of 3.0 shows significant loss in sanguinarine content when the mouthrinse is adjusted to pH 4.5 to pH 5.5. As can be shown in the following example:

EXAMPLE VI

## Stability of Sanguinaria in Rinses

| Wt% Poloxamer 407 | pH | Initial SaCl,*ppm | Aged 3 Months at 40°C SaCl,ppm |
|---|---|---|---|
| 0.1 | 2.7 | 100 | 73 |
| 0.1 | 4.5 | 99 | 58 |
| 0.1 | 5.0 | 109 | 33 |

*Sanguinarine chloride

In determining the reason for the loss of sanguinaria stability at elevated pH's it was unexpectedly found that this decreased stability at the elevated pH's was in part due to a surfactant (poloxamer 407). This nonionic surfactant is a block copolymer of polyoxyethylene and polyoxypropylene. The following data on mouthrinses formulated at pH 6.0 shows that as the concentration of poloxamer is increased in a mouthrinse the stability of sanguinaria decreases.

| Wt. % Poloxamer 407 | 3 Day Stability 40°C SaCl,ppm |
|---|---|
| 0.3 | 94 |
| 0.6 | 49 |
| 0.9 | 20 |

Poloxamer 237 shows improved stability in rinses formulated at elevated pH's as shown by the following data:

| 0.1 Wt.% Poloxamer | ph | Stability at 40°C Initial | SaCl,ppm | Age-Weeks |
|---|---|---|---|---|
| 407 | 4.5 | 101 | 45 | 10 |
| 407 | 5.0 | 106 | 40 | 10 |
| 237 | 4.5 | 92 | 77 | 13 |
| 237 | 5.2 | 95 | 71 | 13 |

It was determined that the poloxamer 407 has a higher level of unsaturation than poloxamer 237 as shown in the following table:

| Surfactant | Unsaturation Meq/mg |
|---|---|
| Poloxamer 407 | 48 |
| Poloxamer 237 | 34 |

Unsaturation was determined by reacting methanolic mercuric acetate with the poloxamer, adding sodium bromide and then titrating the liberated acetic acid with methanolic potassium hydroxide.

EXAMPLE VII

The clinical efficacy of sanguinaria extract is due in part to its antimicrobial activity. Sanguinarine is known to inhibit 98% of the organisms found in dental plaque. (Antimicrobial Agents and Chemotherapy, Apr. 1985, 27:4:663-665). Its MIC is generally 16 PPM or less for these organisms. It is known that sanguinarine extract is absorbed into plaque and onto oral soft and hard tissues (JADA Vol. 108, March 1984, p.338). It has now been discovered that by raising the pH of the mouthrinse from 3.0 to 4.5-5.6 there is an increased uptake of sanguinarine and total benzophenanthridine alkaloids into dental plaque as shown by the following table:

### Sanguinarine and Total Benzophenanthridine Alkaloids Taken Into Wet Plaque from Mouthrinses

| % SaCl | % ZnCl$_2$ | pH | Sanguinarine ug/g wet plaque | Total Benzophenanthridine Alkaloids ug/g plaque |
|---|---|---|---|---|
| 0.03 | 0.2 | 3.2 | 35.6 | 62.7 |
| 0.03 | - | 4.5 | 59.6 | 118.3 |
| 0.03 | 0.2 | 4.5 | 44.2 | 77.6 |
| 0.03 | - | 5.6 | 61.1 | 126.8 |
| 0.03 | 0.2 | 5.6 | 41.5 | 74.4 |

EXAMPLE VIII

In addition to measuring the uptake of sanguinaria extract into plaque, the efficacy of pH 3 and pH 4.5 rinses was measured using a modified saliva glucose test. An initial saliva sample was taken (background) and then subjects rinsed for one minute with a test rinse. The purpose of the back ground saliva sample was to measure shifts in the pH of the sample over time following the glucose challenge. One hour after rinsing with sanguinaria rinses with pH's adjusted to either pH 3.2 or pH 4.5 the saliva of the subjects was again collected under standard conditions. Glucose was then added to the background saliva samples and test saliva samples and the mixtures were incubated for three hours at 37°C. The pH's of these mixtures was monitored at 15, 30, 60, 120, and 300 minutes. Inhibition of salivary glycolysis was measured by subtracting the background pH reading at each timepoint from the test pH reading of the corresponding timepoint. Most of the background saliva samples rapidly decreased in pH after the addition of glucose. Thus, if the rinse treatment provides a protective effect the fall in pH is slow. The larger the differences, therefore, between the test and background pH, the better the inhibition of salivary glycolysis.

The following table shows that a treatment with a pH 4.5 rinse was more effective than treatment with a pH 3.2 rinse. The number of subjects was seven per group. The initial readings were used to adjust the outcome pH levels at each timepoint in order to test for differences between groups. The difference between the test rinses was statistically significant using analysis of covariance (ANCOVA) at a significance level of P .05.

```
         Inhibition of Salivary Glycolysis
         5% Glucose added to Saliva Taken at
         Baseline and One Hour after Rinsing.
                       pH 4.5 Rinse    pH 3.2 Rinse
         Time (Minutes)        pH              pH
              15            1.595           1.465
              30            1.990           1.626
              60            2.148           1.720
             120            2.160           1.523
             300            2.093           1.055
    pH = Sample pH - Background pH at each time period.
```

**Claims**

1.  An oral composition containing benzophenanthridine alkaloid characterized in that it is stabilized by the presence of an orally acceptable buffer system buffered to acid pH.

2.  An oral composition as claimed in Claim 1 characterized in that it is buffered to pH 4.0 to 5.6.

3.  A stable oral rinse composition comprising from about 0.01 to 0.2% by weight of benzophenanthridine alkaloid in an aqueous solution and a buffer system in a quantity sufficient to adjust the pH of said composition to a pH in the range of 4.0 to 5.6.

4.  A composition as claimed in Claim 1, 2 or 3 characterized in that the said buffer system is citrate, acetate, tartrate, succinate, lactate, propionate or phosphate buffer system.

5.  A composition as claimed in any one of Claims 1 to 4 characterized in that it comprises a water-soluble zinc salt.

6.  A composition as claimed in Claim 5 characterized in that the zinc salt is zinc chloride.

7.  A composition as claimed in any one of Claims 1 to 6 characterized in that the said buffer system is selected from (a) sodium citrate and citric acid, (b) sodium acetate and acetic acid, and (c) sodium tartrate and tartaric acid.

8.  A composition as claimed in any one of Claims 1 to 7 characterized in that it further comprises 5 to 25% by weight ethyl alcohol, 2 to 15% by weight of humectant, 0.1 to 3% by weight surface active agent, 0.02 to 1% by weight sweetening agent, and 0.05 to 0.4% by weight flavoring agent.

9.  A composition as claimed in Claim 8 characterized in that the said alkaloid is sanguinarine or chelerythrine.

10. A composition as claimed in any one of Claims 1 to 9 characterized in that it has a pH of about 4.5.

11. A composition as claimed in any one of Claims 1 to 10 characterized in that the said alkaloid is selected from water soluble salts of sanguinarine, chelerythrine, sanguilutine, chelilutine, chelirubine, and sanguirubine; and in that the said buffer system is selected from (a) sodium citrate and citric acid, (b) sodium acetate and acetic acid, and (c) sodium tartrate and tartaric acid.

12. A composition as claimed in any one of the preceding claims in which if a surface active agent is present and if it is a nonionic block copolymer of polyoxyethylene and polyoxypropylene, then it has an unsaturation of less than 45 meq/mg.

**Patentansprüche**

1.  Orale Zusammensetzung, die Benzophenanthridin-Alkaloid enthält, dadurch gekennzeichnet, daß sie durch die Gegenwart eines oral akzeptablen Puffersystems stabilisiert ist, das auf einen sauren pH-Wert puffert.

2.  Orale Zusammensetzung nach Anspruch 1, dadurch gekennzeichnet, daß sie auf einen pH-Wert von 4,0 bis 5,6 gepuffert ist.

3.  Stabile Mundspülzusammensetzung, die etwa 0,01 bis 0,2 Gew.% Benzophenanthridin-Alkaliod in wäßriger Lösung und ein Puffersystem in einer Menge enthält, die ausreicht, den pH-Wert der Zusammensetzung auf einen pH-Wert im Bereich von 4,0 bis 5,6 einzustellen.

4.  Zusammensetzung nach Anspruch 1, 2 oder 3, dadurch gekennzeichnet, daß das Puffersystem ein Citrat-, Acetat-, Tartrat-, Succinat-, Lactat-, Propionat- oder Phosphatpuffersystem ist.

5.  Zusammensetzung nach einem der Ansprüche 1 bis 4, dadurch gekennzeichnet, daß sie ein wasserlösliches Zinksalz umfaßt.

6.  Zusammensetzung nach Anspruch 5, dadurch gekennzeichnet, daß das Zinksalz Zinkchlorid ist.

7.  Zusammensetzung nach einem der Ansprüche 1 bis 6, dadurch gekennzeichnet, daß das Puffersystem ausgewählt ist aus (a) Natriumcitrat und Zitronensäure, (b) Natriumacetat und Essigsäure und (c) Natriumtartrat und Weinsäure.

8.  Zusammensetzung nach einem der Ansprüche 1 bis 7, dadurch gekennzeichnet, daß sie ferner 5 bis 25 Gew.-% Ethylalkohol, 2 bis 15 Gew.-% Feuchthaltemittel, 0,1 bis 3 Gew.-% oberflächenaktives Mittel, 0,02 bis 1 Gew.-% Süßstoff und 0,05 bis 0,4 Gew.-% Geschmacksstoff umfaßt.

9.  Zusammensetzung nach Anspruch 8, dadurch gekennzeichnet, daß das Alkaloid Sanguinarin oder Chelerythrin ist.

10. Zusammensetzung nach einem der Ansprüche 1 bis 9, dadurch gekennzeichnet, daß sie einen pH-Wert von etwa 4,5 aufweist.

11. Zusammensetzung nach einem der Ansprüche 1 bis 10, dadurch gekennzeichnet, daß das Alkaloid ausgewählt ist aus wasserlöslichen Salzen von Sanguinarin, Chelerythrin, Sanguilutin, Chelilutin, Chelirubin und Sanguirubin, und daß das Puffersystem ausgewählt ist aus (a) Natriumcitrat und Zitronensäure, (b) Natriumacetat und Essigsäure und (c) Natriumtartrat und Weinsäure.

12. Zusammensetzung nach einem der vorhergehenden Ansprüche, bei der, wenn ein oberflächenaktives Mittel vorhanden ist und es ein nichtionisches Blockcopolymer aus Polyoxyethylen und Polyoxypropylen ist, es eine Ungesättigtheit von weniger als 45 meq/mg aufweist.

**Revendications**

1.  Composition orale contenant un alcaloïde benzophénanthridine, caractérisée en ce qu'elle est stabilisée en présence d'un système tampon, acceptable pour l'administration orale, à un pH acide.

2.  Composition orale selon la revendication 1, caractérisée en ce qu'elle est tamponnée à un pH de 4,0 à 5,6.

3.  Composition orale de rinçage stable, comprenant d'environ 0,01 à 0,2% en poids d'alcaloïde benzophénanthridine en solution aqueuse et un système tampon, en quantité suffisante pour ajuster le pH de ladite composition à un domaine de pH de 4,0 à 5,6.

4. Composition selon la revendication 1, 2 ou 3, caractérisée en ce que ledit système tampon est un système tampon à base de citrate, d'acétate, de tartrate, de succinate, de lactate, de propionate ou de phosphate.

5. Composition selon l'une quelconque des revendications 1 à 4, caractérisée en ce qu'elle comprend un sel de zinc soluble dans l'eau.

6. Composition selon la revendication 5, caractérisée en ce que le sel de zinc est un chlorure de zinc.

7. Composition selon l'une quelconque des revendications 1 à 6, caractérisée en ce que ledit système tampon est choisi parmi (a) le citrate de sodium et l'acide citrique, (b) l'acétate de sodium et l'acide acétique et (c) le tartrate de sodium et l'acide tartrique.

8. Composition selon l'une quelconque des revendications 1 à 7, caractérisée en ce qu'elle comprend aussi 5 à 25% en poids d'alcool éthylique, 2 à 15% en poids d'humidifiant, 0,1 à 3% en poids d'agent tensioactif, 0,02 à 1% en poids d'agent édulcorant et 0,05 à 0,4% en poids d'agent aromatisant.

9. Composition selon la revendication 8, caractérisée en ce que ledit alcaloïde est la sanguinarine ou la chélérythrine.

10. Composition selon l'une quelconque des revendications 1 à 9, caracterisée en ce qu'elle possède un pH d'environ 4,5.

11. Composition selon l'une quelconque des revendications 1 à 10, caractérisée en ce que ledit alcaloïde est choisi parmi les sels hydrosolubles de sanguinarine, de chélérythrine, de sanguilutine, de chélilutine, de chélirubine et de sanguirubine; et en ce que ledit système tampon est choisi parmi (a) le citrate de sodium et l'acide citrique, (b) l'acétate de sodium et l'acide acétique et (c) le tartrate de sodium et l'acide tartrique.

12. Composition selon l'une quelconque des revendications précédentes, dans laquelle, si un agent tensioactif est présent et s'il s'agit d'un copolymère séquencé non ionique de polyoxyéthylène et de polyoxypropylène, il possède alors une insaturation inférieure à 45 méq/mg.